# FASCICULE DE BREVET EUROPEEN

(11) **EP 0 645 183 B1**
(45) Date de publication et mention de la délivrance du brevet: **30.07.1997**
(21) Numéro de dépôt: 94401980.1
(22) Date de dépôt: 06.09.1994
(51) Int. Cl.: B01J 27/053, C07C 2/62

(54) **Catalyseur d'alkylation d'isoparaffine C4-C5 par au moins une oléfine C3-C6**
Katalysator zur Alkylierung von einem C4-C5 Isoparaffin mit mindestens einem C3-C6 Olefin
Catalyst for alkylating a C4-C5 isoparaffin with at least a C3-C6 olefin

(30) Priorité: 10.09.1993 FR 9310896; 14.12.1993 FR 9314994
(43) Date de publication de la demande: 29.03.1995
(73) Titulaire: INSTITUT FRANCAIS DU PETROLE, 92502 Rueil-Malmaison (FR)
(72) Inventeur: Benazzi, Eric, F-78360 Montesson (FR); Joly, Jean-François, F-75003 Paris (FR); Chaigne, Frédéric, F-85230 Bauvoir S/Mer (FR); Bernhard, Jean-Yves, F-91540 Mennecy (FR); Marcilly, Christian, F-78800 Houilles (FR)

(56) Documents cités:
- EP-A- 0 539 277
- EP-A- 0 605 279
- DE-A- 2 510 095
- US-A- 5 233 119

## Description

La présente invention concerne un catalyseur comprenant de la silice et une phase acide comportant de l'acide sulfurique et le composé HB(HSO₄)₄ , la silice ayant été imprégnée par ladite phase acide. L'invention concerne aussi la préparation et l'utilisation dudit catalyseur en alkylation catalytique d'isoparaffine (isobutane et/ou isopentane) en présence d'au moins une oléfine comportant de 3 à 6 atomes de carbone par molécule.

Pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation d'isoparaffine (isobutane et/ou isopentane) par au moins une oléfine contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent généralement soit l'acide sulfurique soit l'acide fluorhydrique comme catalyseur. Dans ces procédés le catalyseur acide constitue une phase liquide qui est mise en contact avec le mélange isobutane-oléfine(s) liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

Pour catalyser les réactions d'alkylation des isoparaffines par les oléfines, on a déjà proposé de mettre au point des catalyseurs acides à partir de nombreux solides acides de natures différentes. Parmi les familles de catalyseurs acides on peut citer les tamis moléculaires, les résines macroréticulaires éventuellement associées avec BF₃, les acides de Lewis et/ou de Brönsted déposés sur divers supports inorganiques, les alumines chlorées, les graphites intercalés par des acides de Lewis et/ou de Brönsted et les anions déposés sur supports oxydes tels que ZrO₂/SO₄. Ces solides conduisent à la production d'isoparaffines ramifiées mais souffrent de plusieurs défauts majeurs, parmi lesquels on peut citer l'utilisation de rapports molaires isobutane/oléfine souvent très élevés pour limiter l'importance des réactions secondaires et la faible stabilité dans le temps de l'activité catalytique (inhibition du catalyseur par dépôt d'oligomères insaturés) ; ces catalyseurs doivent alors être fréquemment régénérés. De plus, la faible acidité de certains solides acides, tels que les tamis moléculaires par exemple, impose l'utilisation de températures de réaction élevées ce qui est préjudiciable à l'obtention d'hydrocarbures d'indices d'octane élevés.

La demande de brevet européen EP-A-0539277 décrit un catalyseur contenant de la silice et une phase acide solide comprenant de l'acide sulfurique la silice selon ladite demande de brevet est telle que son volume poreux est compris entre 0,005 et 1,5 cm³/g, et sa surface spécifique est comprise entre 0,01 et 1 500 m²/g. Ladite phase acide comprend éventuellement un additif choisi dans le groupe formé par H₃PO₄, B(OH)₃, BF₄H, FSO₃H, CF₃CO₂H, SbF₅, CF₃SO₃H et SO₃.

La demande de brevet français FR-A-2 687 935 décrit un catalyseur comprenant une silice de surface spécifique comprise entre 0,01 et 1500 m²/g et de volume poreux total compris entre 0,005 et 1,5 cm³/g et une phase acide à l'état solide comprenant au moins l'acide sulfurique et l'anhydride sulfurique, la silice ayant été imprégnée par ladite phase acide. Ladite phase acide contient éventuellement de l'acide borique B(OH)₃, la teneur pondérale en acide borique étant comprise entre 0,01% et 50%.

La présente invention concerne un catalyseur comprenant de la silice et une phase acide comportant de l'acide sulfurique et le composé HB(HSO₄)₄, la silice ayant été imprégnée par ladite phase acide, ledit catalyseur étant tel qu'il est constitué essentiellement de particules de diamètre moyen compris entre 0,1 et 150 µm (1 µm = 10⁻⁶m) de préférence entre 5 et 110 µm et de manière encore plus préférée entre 5 et 80 µm, en ce que sa teneur pondérale en phase acide est supérieure à 40 %, de préférence supérieure à 70 %, en ce que la silice, avant son imprégnation par ladite phase acide, possède un volume poreux total compris entre 0,5 et 6 cm³/g, de préférence compris entre 0,6 et 6 cm³/g, et de façon encore plus préférée compris entre 1,5 et 6 cm³/g, et en ce que ladite phase acide comprend (en % poids) entre 0,4 et 68,8 %, de préférence entre 0,4 et 60 %, du composé HB(HSO₄)₄ et entre 31,2 et 99,6 %, de préférence entre 40 et 99,6 %, du composé H₂SO₄, ladite phase acide étant caractérisée par le fait qu'elle ne contient pas d'anhydride sulfurique (SO₃) ni d'acide borique [B(OH)₃].

En particulier, ladite phase acide ne comprend pas d'anhydride sulfurique non associé, c'est-à-dire n'ayant pas réagi avec l'acide borique, ni d'acide borique non associé, c'est-à-dire n'ayant pas réagi avec l'anhydride sulfurique.

L'invention concerne aussi la préparation et l'utilisation dudit catalyseur en alkylation catalytique d'au moins une isoparaffine choisi dans le groupe formé par l'isobutane et l'isopentane (c'est-à-dire l'isobutane et/ou l'isopentane : l'isobutane, ou l'isopentane, ou l'isobutane et l'isopentane) en présence d'au moins une oléfine comportant de 3 à 6 atomes de carbone par molécule.

Le catalyseur selon la présente invention conduit de façon surprenante à des performances catalytiques améliorées par rapport à celles décrites dans la demande de brevet européen EP-A-0539277 et aussi par rapport à celles décrites dans la demande de brevet français FR-A-2 687 935.

Le composé HB(HSO₄)₄ compris dans la phase acide du catalyseur selon l'invention peut être obtenu par toutes les méthodes connues de l'homme du métier. Par exemple et à titre non limitatif, on peut citer la méthode préférée selon l'invention qui consiste à faire réagir 1 mole d'acide borique B(OH)₃ avec 3 moles d'anhydride sulfurique SO₃ et 1 mole d'acide sulfurique H₂SO₄ pour obtenir 1 mole du composé HB(HSO₄)₄.

La silice utilisée comme support peut contenir des impuretés comme par exemple les oxydes, les alcalins, les alcalino-terreux, les composés d'aluminium ou toute autre impureté connue de l'homme du métier, la quantité totale de ces impuretés n'excédant généralement pas 2 % en poids par rapport à la silice.

La silice est généralement telle que, avant son imprégnation par ladite phase acide, sa surface spécifique est comprise entre 0,1 et 1 500 m²/g. De plus, ladite silice est généralement constituée essentiellement de particules de diamètre moyen compris entre 0,1 et 150 µm, de préférence entre 5 et 110 µm et de manière encore plus préférée entre 5 et 80 µm.

La phase acide occupe généralement entre 80 et 100 % du volume poreux total de la silice, et de préférence entre 90 et 100 % dudit volume poreux.

Le procédé de préparation du catalyseur selon l'invention comprend généralement au moins deux étapes. Dans une première étape la silice est calcinée à une température supérieure à 50 °C, de préférence supérieure à 80 °C et de manière encore plus préférée comprise entre 150 et 600°C, par exemple égale à environ 500° C. La durée de cette étape de calcination est habituellement comprise entre 10 minutes et 50 heures, de préférence entre 15 minutes et 25 heures. La calcination est généralement effectuée en présence d'air sec ou de mélange air sec/azote, de débit compris entre 0,001 et 10 l/h/g, de préférence entre 0,1 et 5 l/h/g. La deuxième étape consiste en l'imprégnation de ladite silice calcinée par ladite phase acide. Pour réaliser cette étape on peut utiliser toutes les techniques bien connues de l'homme du métier. A ce procédé de préparation peut s'ajouter une étape de préparation de la phase acide, préalable à l'étape d'imprégnation.

Le catalyseur selon l'invention ainsi préparé n'a subi aucune calcination postérieure à l'étape d'imprégnation. Lorsqu'il est utilisé en alkylation d'isoparaffine(s) par au moins une oléfine, il ne subit avant son utilisation aucune calcination et ainsi entre l'étape d'imprégnation et ladite utilisation, il n'a subi aucune calcination. Le catalyseur selon l'invention ainsi préparé est donc immédiatement prêt à l'emploi.

Le catalyseur selon la présente invention est mis en oeuvre dans un procédé qui permet de réaliser dans les meilleures conditions la réaction d'alkylation de l'isoparaffine par au moins une oléfine. En particulier, ladite réaction étant caractérisée par une forte exothermicité (environ 83,6 kJ/mol de butène transformé si l'oléfine est le butène et si l'isoparaffine est l'isobutane), la mise en oeuvre du catalyseur selon la présente invention permet d'obtenir une bonne homogénéité de température et de concentration en réactifs.

Dans le procédé d'alkylation de l'(les) isoparaffine(s) utilisant le catalyseur selon la présente invention, les conditions opératoires, et plus particulièrement la température et la pression, sont généralement choisies de façon à ce que le mélange constitué par l'(les) isoparaffine(s), l'(les) oléfine(s) et les produits de la réaction soit liquide. De plus, il est important que le catalyseur soit immergé dans ledit liquide de façon à assurer un bon contact liquide-solide.

Le catalyseur selon l'invention est avantageusement mis en oeuvre dans la zone réactionnelle d'alkylation de l'isobutane et/ou de l'isopentane avec au moins une oléfine comprenant de 3 à 6 atomes de carbone par molécule, en phase liquide et en mélange avec l'isoparaffine ou un mélange d'isoparaffines. Le catalyseur selon l'invention peut être mis en oeuvre en lit expansé, en zone réactionnelle presque parfaitement agitée ou en lit circulant, et de préférence il est mis en oeuvre dans un procédé qui utilise une phase liquide continue, le catalyseur étant utilisé sous forme de suspension par exemple selon les deux mises en oeuvre décrites ci-après.

Dans le cas où le catalyseur est utilisé sous forme d'une suspension, on peut utiliser dans une première mise en oeuvre une zone réactionnelle à mélange presque parfait, c'est-à-dire à mélange parfait ou s'en rapprochant (cuve agitée ou Grignard), utilisant au moins un moyen d'agitation par exemple au moins une hélice, de façon à obtenir une agitation suffisante du catalyseur en suspension dans la phase liquide hydrocarbonée, laquelle comprend généralement l'isoparaffine (isobutane et/ou isopentane), au moins une oléfine, éventuellement au moins un diluant inerte (par exemple le propane et le normal butane) et les produits de la réaction d'alkylation. La charge à convertir, constituée d'isobutane et/ou d'isopentane et d'au moins une oléfine, peut être par exemple introduite sous forme liquide en au moins un point au sein de la phase liquide hydrocarbonée présente dans la zone réactionnelle.

Une deuxième mise en oeuvre du catalyseur selon la présente invention en suspension dans une phase hydrocarbonée est le lit mobile à co-courant ou lit circulant. Dans cette mise en oeuvre le catalyseur en suspension dans la phase liquide hydrocarbonée, comprenant généralement l'isobutane et/ou l'isopentane, au moins une oléfine, éventuellement au moins un diluant inerte (par exemple le propane ou le normal butane) et les produits de la réaction d'alkylation, circule de bas en haut dans la zone réactionnelle. L'ensemble constitué par la suspension de catalyseur dans la phase hydrocarbonée circule ensuite au travers d'au moins un échangeur de chaleur et d'au moins une pompe, avant d'être de nouveau introduit à l'entrée de la zone réactionnelle. La charge à convertir, constituée d'isobutane et/ou d'isopentane et d'au moins une oléfine, est introduite soit sous forme liquide, soit sous forme gazeuse en au moins un point de la zone réactionnelle.

Dans les deux types de mises en oeuvre décrites précédemment, l'isoparaffine (isobutane et/ou isopentane) n'ayant pas été convertie ou ayant été introduite en excès par rapport à la stoechiométrie de la réaction est généralement recyclée après séparation de l'alkylat soit par introduction directe dans la zone réactionnelle soit par mélange avec la charge à convertir.

Le mélange isoparaffine(s)-oléfine(s) est généralement introduit dans la zone réactionnelle à une vitesse spatiale horaire, exprimée en poids d'oléfine introduite par unité de poids du catalyseur et par heure (pph) comprise entre 0,001 et 10 h⁻¹ et de préférence comprise entre 0,002 et 2 h⁻¹. Ledit mélange peut aussi être réalisé à l'intérieur de la zone réactionnelle. Dans tous les cas le mélange ainsi constitué est dans la zone réactionnelle dans des conditions de pression et de température telles que le mélange d'hydrocarbures reste liquide sur le catalyseur.

La température de réaction est généralement inférieure à +10° C, de préférence inférieure à 0 °C et de manière souvent plus préférée inférieure à -3° C. La pression de la zone réactionnelle est suffisante pour maintenir les hydrocarbures à l'état liquide dans ladite zone.

Afin de limiter les réactions secondaires, on peut utiliser un excès d'isoparaffine(s) par rapport à l' (les) oléfine(s). A titre d'exemple, dans le cas de l'alkylation de l'isobutane par un butène, l'isobutane peut être introduit pur dans la charge ou sous la forme d'un mélange de butanes contenant par exemple au moins 40% d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 15.

Lorsque la nature du catalyseur et les conditions de réaction sont judicieusement choisies (en particulier la température), le catalyseur selon l'invention permet la production de produits d'alkylation d'au moins une isoparaffine par au moins une oléfine qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comportant 70 à 98 % en moles de triméthylpentanes.

Un autre avantage du catalyseur selon la présente invention est la possibilité d'alkyler, à basse température, l'isoparaffine avec des mélanges d'oléfines comportant de 3 à 6 atomes de carbone par molécule, où la proportion d'oléfines comportant plus de 4 atomes de carbone par molécule est très importante.

Les exemples suivants illustrent l'invention sans toutefois en limiter la portée.

### Exemple 1

### Préparation du catalyseur 1 conforme à l'invention

On active 14 g de silice macroporeuse, de surface spécifique égale à 27 m²/g et de volume poreux égal à 1 cm³/g, de diamètre moyen des particules égal à 110 µm, par calcination sous air pendant 4 heures à 500°C. Le solide ainsi activé est conservé sous argon. Puis on prépare 10 cm³ d'un mélange constitué de 80% poids d'acide sulfurique (99,99%) et de 20 % poids de d'anhydride sulfurique. Ensuite on ajoute 0,993g d'acide borique aux 10 cm³ du mélange préparé ci-dessus pour obtenir 20,28 g de phase acide. Suite à la réaction de l'anhydride sulfurique et de l'acide borique en présence d'acide sulfurique et respectivement dans les rapports molaires 3/1/1 (c'est-à-dire 1 mole d'acide borique avec 3 moles d'anhydride sulfurique et 1 mole d'acide sulfurique), on obtient une phase acide qui contient le composé HB(HSO₄)₄ en solution dans H₂SO₄ et comportant 31,69% poids de HB(HSO₄)₄ et 68,31% poids de H₂SO₄.

On procède alors à une imprégnation à sec de 11 g du solide calciné par 14,31g du mélange décrit ci-dessus. Le catalyseur 1 ainsi obtenu contient 14,31g de phase acide et 11 g de silice, soit une teneur pondérale en phase acide de 56,5 %; il est conservé sous atmosphère d'argon à -18° C.

### Alkylation de l'isobutane par le butène-1 avec le catalyseur 1

On introduit 20 g du catalyseur 1 préparé selon la méthode décrite dans l'exemple 1 dans un réacteur en verre du type Fischer & Porter d'un volume de 360 cm³ préalablement purgé sous débit d'argon. Le réacteur contenant le catalyseur est alors fermé puis mis sous vide primaire, puis il est refroidi à la température de -20°C.

72 cm³ d'isobutane sont alors ajoutés dans le réacteur contenant le catalyseur sous agitation, ledit réacteur étant immergé dans un bain froid à -20 °C. On laisse sous agitation (hélice) le système catalyseur + isobutane pendant une durée de 30 minutes afin d'homogénéiser la température.

On ajoute régulièrement 100 cm³ d'un mélange constitué de 24% en volume de butène-1 et de 76% en volume d'isobutane, pendant une durée totale de 10 heures, la température du réacteur étant maintenue à -15 °C pendant toute la durée de l'injection.

Après réaction, la phase hydrocarbure est soutirée du réacteur, l'isobutane est évaporé lentement. On recueille l'alkylat qui est analysé par chromatographie en phase vapeur ; sa composition pondérale est donnée dans le tableau 1.

### Exemple 2

### Préparation du catalyseur 2 (selon la demande de brevet français FR-A-2 687 935)

Pour préparer le catalyseur 2, on utilise 11g de la même silice macroporeuse que celle utilisée pour la préparation du catalyseur 1, les conditions de calcination sont identiques. Le solide ainsi activé est conservé sous argon. Puis on prépare 7 cm³ d'un mélange constitué de 80% poids d'acide sulfurique (99,99%) et de 20 % poids d'anhydride sulfurique. Ensuite aux 7 cm³ du mélange préparé ci-dessus sont ajoutés 0,81g d'acide borique pour obtenir 14,31g de phase acide. Suite à la réaction de l'anhydride sulfurique et de l'acide borique en présence d'acide sulfurique et respectivement dans les rapports molaires 3/1/1 on obtient une phase acide qui contient le composé HB(HSO₄)₄ en solution dans H₂SO₄ et comportant 31,42% poids de HB(HSO₄)₄, 67,76% poids de H₂SO₄ et 0,82% poids de B(OH)₃.

On procède alors à une imprégnation à sec de 11 g du solide calciné par la totalité du mélange décrit ci-dessus. Le catalyseur 2 ainsi obtenu contient 14,31g de phase acide et 11 g de silice, soit une teneur pondérale en phase acide de 56,5 %; il est conservé sous atmosphère d'argon à -18° C.

### Alkylation de l'isobutane par le butène-1 avec le catalyseur 2

On répète le test catalytique d'alkylation de l'isobutane par le butène-1 dans les mêmes conditions expérimentales que celles décrites dans l'exemple 1 mais au lieu de d'ajouter régulièrement 100 cm³ d'un mélange constitué de 24% en volume de butène-1 et de 76% en volume d'isobutane, pendant une durée totale de 10 heures, ce sont 50 cm³ du même mélange qui sont ajoutés en 10 heures soit un débit de réactifs et donc de butène-1 deux fois plus faible que dans l'exemple 1 avec le catalyseur 1. Les résultats sont rassemblés dans le tableau 1.

### Exemple 3

### Préparation du catalyseur 3 non conforme à l'invention

Pour préparer le catalyseur 3, on utilise 11g de la même silice macroporeuse que celle utilisée pour la préparation des catalyseurs 1 et 2, les conditions de calcination étant identiques. Le solide ainsi activé est conservé sous argon. Puis on prépare 30 g d'un mélange constitué de 80 % poids d'acide sulfurique (100 %) et de 20 % poids de d'anhydride sulfurique.

Ensuite on ajoute 1,42 g d'acide borique aux 30 g du mélange préparé ci-dessus afin d'obtenir 31,42 g de phase acide. Suite à la réaction de l'anhydride sulfurique et de l'acide borique en présence d'acide sulfurique et respectivement dans les rapports molaires 3/1/1 (c'est-à-dire 1 mole d'acide borique avec 3 moles d'anhydride sulfurique et 1 mole d'acide sulfurique), on obtient une phase acide qui comportant 29,3 % poids de composé HB(HSO₄)₄, 69,2 % poids d'acide sulfurique et 1,5 % poids d'anhydride sulfurique.

On procède alors à une imprégnation à sec de 11 g du solide calciné par 14,31 g mélange décrit ci-dessus. Le catalyseur 3 ainsi obtenu contient 14,31 g de phase acide et 11 g de silice, soit une teneur pondérale en phase acide de 56,5 %; il est conservé sous atmosphère d'argon à -18° C.

### Alkylation de l'isobutane par le butène-1 avec le catalyseur 3

On répète le test catalytique d'alkylation de l'isobutane par le butène-1 dans les mêmes conditions expérimentales que celles décrites dans l'exemple 2. Les résultats sont rassemblés dans le tableau 1.

**Tabeau 1**

| **Comparaison des catalyseurs 1,2 et 3** | | | |
|---|---|---|---|
| Composition alkylat (% poids) | Catalyseur 1 (conforme à l'invention) | Catalyseur 2 (non conforme à l'invention ; B(OH)₃ libre) | Catalyseur 3 5non conforme à l'invention ; SO₃ libre |
| % C₅-C₇ | 2,0 | 2,4 | 6,2 |
| % C₈ | 95,2 | 94,6 | 85,5 |
| % C₉⁺ | 2,8 | 3 | 8,3 |

Ce tableau met en évidence l'intérêt qu'il y a à travailler avec le catalyseur selon l'invention comprenant une phase acide comportant de l'acide sulfurique et le composé HB(HSO₄)₄, et sans acide borique ni anhydride sulfurique. En effet, la présence du composé HB(HSO₄)₄ dans la phase acide en l'absence de SO₃ et de B(OH)₃ permet d'obtenir un catalyseur 1 selon l'invention plus actif, puisque travaillant avec un débit de réactifs (butène-1 et isobutane) c'est-à-dire avec un débit de butène-1 deux fois plus grand que celui utilisé dans le cas des catalyseurs 2 et 3 qui contiennent respectivement de l'acide borique B(OH)₃ et de l'anhydride sulfurique SO₃. Le catalyseur 1 selon l'invention, dans des conditions de test plus sévères (débit de butène-1, deux fois plus grand), est aussi plus sélectif comme l'indique le tableau 1.

### Exemple 4

### Préparation du catalyseur 4 conforme à l'invention

15 grammes de silice de volume poreux total égal à 2,6 cm³/g, de surface spécifique égale à 420 m²/g et de diamètre moyen des particules égal à 75 µm sont activés par chauffage sous débit de N₂ sec à 500°C durant 12 heures. La silice ainsi activée est conservée sous azote sec. Puis, à 100 g d'une solution d'anhydride sulfurique dans l'acide sulfurique, contenant 11,5% poids d'anhydride sulfurique et 88,5% poids d'acide sulfurique sont ajoutés 2,97 g d'acide borique afin d'obtenir 102,97 g de phase acide. Suite à la réaction de l'anhydride sulfurique et de l'acide borique en présence d'acide sulfurique et respectivement dans les rapports molaires 3/1/1, on obtient une phase acide qui contient le composé HB(HSO₄)₄ en solution dans H₂SO₄ et comprenant 18,6% poids de HB(HSO₄)₄ et 81,4% poids de H₂SO₄.

On procède alors à une imprégnation à sec de 10 g de la silice activée ci-dessus par 47,5 g de la phase décrite ci-avant. Le solide ainsi obtenu, appelé catalyseur 4, possède une teneur pondérale en phase acide égale à 82,6% poids. Il est conservé à l'abri de l'humidité sous argon à - 18° C.

### Alkylation de l'isobutane par le butène-1 avec le catalyseur 4

On introduit 20 g du catalyseur 4 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm³ d'isobutane sont alors ajoutés dans le réacteur contenant le catalyseur sous agitation (hélice), ledit réacteur étant immergé dans un bain froid à -5°C. On laisse sous agitation le système catalyseur + isobutane pendant une durée de 30 minutes afin d'homogénéiser la température.

On ajoute régulièrement 5 g de butène-1 par heure pendant une durée totale de 6 heures, la température du réacteur est maintenu à -5 °C pendant toute la durée de l'injection.

Après réaction, la phase hydrocarbure est soutirée du réacteur, puis est analysée par chromatographie en phase vapeur. Sa composition pondérale est donnée dans le tableau 2. La conversion de l'oléfine est de 100 %.

### Exemple 5

### Préparation du catalyseur 5 non conforme à l'invention

Pour préparer le catalyseur 5, on utilise 10g de la même silice que celle utilisée pour la préparation du catalyseur 4, les conditions de calcination étant identiques. Le solide ainsi activé est conservé sous argon. Puis on procède à l'imprégnation à sec de 10 g de ladite silice activée par 47,5 g d'une solution d'acide sulfurique à 99,9 % poids. Le solide ainsi obtenu, appelé catalyseur 5, possède une teneur pondérale en phase acide égale à 82,6% poids. Il est conservé à l'abri de l'humidité sous argon à - 18° C

### Alkylation de l'isobutane par le butène-1 avec le catalyseur 5

On répète le test catalytique d'alkylation de l'isobutane par le butène-1 dans les mêmes conditions expérimentales que celles décrites dans l'exemple 4. Les résultats sont rassemblés dans le tableau 2.

**Tableau 2**

| **Comparaison des catalyseurs 4 et 5** | | |
|---|---|---|
| Composition alkylat (% poids) | Catalyseur 4 (conforme à l'invention) | Catalyseur 5 (non conforme à l'invention) |
| % C₅-C₇ | 5,3 | 9,1 |
| % C₈ | 91,6 | 82,9 |
| % C₉⁺ | 3,1 | 8,0 |

Ce tableau met en évidence l'intérêt qu'il y a à travailler avec un catalyseur comprenant une phase acide comportant de l'acide sulfurique et le composé HB(HSO₄)₄. En effet, la présence du composé HB(HSO₄)₄ permet d'obtenir un catalyseur 4 selon l'invention plus sélectif comme l'indique le tableau 2.

### Exemple 6

### Préparation du catalyseur 6 conforme à l'invention

15 grammes de silice de volume poreux total égal à 1,85 cm³/g de surface spécifique égale à 186 m²/g et de diamètre moyen des particules égal à 35 µm, sont activés par chauffage sous débit d'azote sec à 500°C durant 12 heures. La silice ainsi activée est conservée sous azote sec. Puis, à 100 g d'une solution d'anhydride sulfurique dans l'acide sulfurique, contenant 25,9% poids d'anhydride sulfurique et 74,1% poids d'acide sulfurique sont ajoutés 6,715 g d'acide borique afin d'obtenir 106,715 g de phase acide. Suite à la réaction de l'anhydride sulfurique et de l'acide borique en présence d'acide sulfurique et respectivement dans les rapports molaires 3/1/1, on obtient une phase acide qui contient le composé HB(HSO₄)₄ en solution dans H₂SO₄ et comprenant 40,5% poids de HB(HSO₄)₄ et 59,5% poids de H₂SO₄.

On procède alors à une imprégnation à sec de 10 g de la silice activée ci-dessus par 34 g de la phase décrite ci-avant. Le solide ainsi obtenu, appelé catalyseur 6, possède une teneur pondérale en phase acide égale à 77,3% poids. II est conservé à l'abri de l'humidité sous argon à - 18° C.

### Alkylation de l'isobutane par le butène-1 avec le catalyseur 6

### Test d'alkylation 6A

On introduit 20 g du catalyseur 6 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm³ d'isobutane sont alors ajoutés dans le réacteur contenant le catalyseur sous agitation (hélice), ledit réacteur étant immergé dans un bain froid à -5°C. On laisse sous agitation le système catalyseur + isobutane pendant une durée de 30 minutes afin d'homogénéiser la température.

On ajoute régulièrement 4 g de butène-1 par heure pendant une durée totale de 24 heures, la température du réacteur est maintenu à -5 °C pendant toute la durée de l'injection.

Après réaction, la phase hydrocarbure est soutirée du réacteur, puis est analysé par chromatographie en phase vapeur. Sa composition pondérale est donnée dans le tableau 3. La conversion de l'oléfine est de 100 %.

### Test d'alkylation 6B

On introduit 20 g du catalyseur 6, préparé selon la méthode décrite ci-dessus, dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm³ d'isobutane sont alors ajoutés dans le réacteur contenant le catalyseur sous agitation (hélice), ledit réacteur étant immergé dans un bain froid à -5°C. On laisse sous agitation le système catalyseur + isobutane pendant une durée de 30 minutes afin d'homogénéiser la température.

On ajoute régulièrement 20g de butène-1 par heure pendant une durée totale de 6 heures, la température du réacteur est maintenu à -5 °C pendant toute la durée de l'injection.

Ainsi le test d'alkylation 6B se distingue du test d'alkylation 6A en ce que le débit de butène-1 est 5 fois plus important dans le test d'alkylation 6B.

Après réaction, la phase hydrocarbure est soutirée du réacteur, puis est analysé par chromatographie en phase vapeur. Sa composition pondérale est donnée dans le tableau 3. La conversion de l'oléfine est de 100 %.

### Exemple 7

### Préparation du catalyseur 7 non conforme à l'invention

Pour préparer le catalyseur 7, on utilise 10g de la même silice que celle utilisée pour la préparation du catalyseur 6, les conditions de calcination sont identiques. Le solide ainsi activé est conservé sous argon. Puis on procède à l'imprégnation à sec de 10 g de la silice activée ci-dessus par 34 g d'une solution d'acide sulfurique (H₂SO₄) à 99,9% poids. Le solide ainsi obtenu, appelé catalyseur 7, possède une teneur pondérale en phase acide égale à 77,3% poids. Il est conservé à l'abri de l'humidité sous argon à -18° C.

### Alkylation de l'isobutane par le butène-1 avec le catalyseur 7

### Test d'alkylation 7A

On introduit 20 g du catalyseur 7, préparé selon la méthode décrite ci-dessus, dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm³ d'isobutane sont alors ajoutés dans le réacteur contenant le catalyseur sous agitation (hélice), ledit réacteur étant immergé dans un bain froid à -5°C. On laisse sous agitation le système catalyseur + isobutane pendant une durée de 30 minutes afin d'homogénéiser la température.

On ajoute régulièrement 4g de butène-1 par heure pendant une durée totale de 24 heures, la température du réacteur est maintenu à -5 °C pendant toute la durée de l'injection. Il s'agit des mêmes conditions opératoires que celles utilisées pour le test d'alkylation 6A.

Après réaction, la phase hydrocarbure est soutirée du réacteur, puis est analysé par chromatographie en phase vapeur. Sa composition pondérale est donnée dans le tableau 3. La conversion de l'oléfine est de 100 %.

### Test d'alkylation 7B

On répète le test d'alkylation de l'isobutane par le butène-1 avec le catalyseur 7 dans les mêmes conditions expérimentales que celles décrites dans l'exemple 6 pour le test d'alkylation 6B. Les résultats sont rassemblés dans le tableau 3.

**Tableau 3**

| **Comparaison des catalyseurs 6 et 7** | | | | |
|---|---|---|---|---|
| Composition alkylat (% poids) | Catalyseur 6 (conforme à l'invention) Test d'alkylation 6A | Catalyseur 7 (non conforme à l'invention) Test d'alkylation 7A | Catalyseur 6 (conforme à l'invention) Test d'alkylation 6B | Catalyseur 7 (non conforme à l'invention) Test d'alkylation 7B |
| % C₅-C₇ | 4 | 8,4 | 8,7 | - |
| % C₈ | 92,9 | 84,7 | 81,6 | - |
| % C₉⁺ | 3,1 | 6,9 | 9,7 | C₉⁺ > 80 |

Ce tableau met en évidence l'intérêt qu'il y a à travailler avec un catalyseur selon l'invention comprenant une phase acide comportant de l'acide sulfurique et le composé HB(HSO₄)₄. En effet, la présence du composé HB(HSO₄)₄ permet d'obtenir un catalyseur 6 selon l'invention plus sélectif comme l'indique, dans le tableau 3, le test d'alkylation 6A par rapport au test d'alkylation 7A réalisé avec le catalyseur 7, non conforme à l'invention, dans les mêmes conditions opératoires.

Par ailleurs, la comparaison tests d'alkylation 6B et 7B, effectués dans les mêmes conditions expérimentales, montre que le catalyseur 6 conforme à l'invention est plus actif et sélectif que le catalyseur 7, non conforme à l'invention, qui conduit majoritairement à la formation de composés lourds C₉⁺.

## Revendications

1. Catalyseur comprenant de la silice et une phase acide comportant de l'acide sulfurique et le composé HB(HSO₄)₄, la silice ayant été imprégnée par ladite phase acide, ledit catalyseur étant tel qu'il est constitué essentiellement de particules de diamètre moyen compris entre 0,1 et 150 µm, en ce que sa teneur pondérale en phase acide est supérieure à 40 %, en ce que la silice, avant son imprégnation par ladite phase acide, possède un volume poreux total compris entre 0,5 et 6 cm³/g, en ce que ladite phase acide comprend (en % poids) entre 0,4% et 68,8% du composé HB(HSO₄)₄ et entre 31,2% et 99,6% du composé H₂SO₄, ladite phase acide étant caractérisée par le fait qu'elle ne contient pas d'anhydride sulfurique ni d'acide borique.

2. Catalyseur selon la revendication 1 tel qu'il est constitué essentiellement de particules de diamètre moyen compris entre 5 et 110 µm.

3. Catalyseur selon l'une des revendications 1 ou 2 tel que la silice, avant son imprégnation par ladite phase acide, possède un volume poreux total compris entre 0,6 et 6 cm³/g.

4. Catalyseur selon l'une des revendications 1 à 3 tel que la silice, avant son imprégnation par ladite phase acide, possède un volume poreux total compris entre 1,5 et 6 cm³/g.

5. Catalyseur selon l'une des revendications 1 à 4 tel que la teneur pondérale en ladite phase acide est supérieure à 70 %.

6. Préparation du catalyseur selon l'une des revendications 1 à 5 comprenant au moins deux étapes, la première étape étant telle que la silice est calcinée à une température supérieure à 50°C pour une durée comprise entre 10 minutes et 50 heures, et la deuxième étape étant telle que ladite silice calcinée est imprégnée par ladite phase acide.

7. Préparation selon la revendication 6 dans laquelle le composé HB(HSO₄)₄ est obtenu en faisant réagir 1 mole d'acide borique avec 3 moles d'anhydride sulfurique et 1 mole d'acide sulfurique.

8. Utilisation du catalyseur selon l'une des revendications 1 à 5 ou préparé selon l'une des revendications 6 ou 7 en alkylation catalytique d'au moins une isoparaffine choisi dans le groupe formé par l'isobutane et l'isopentane en présence d'au moins une oléfine comportant de 3 à 6 atomes de carbone par molécule dans laquelle la température de réaction est inférieure à +10° C et la pression de la zone réactionnelle est suffisante pour maintenir les hydrocarbures à l'état liquide dans ladite zone.

9. Utilisation selon la revendication 8 dans laquelle le catalyseur est mis en oeuvre dans une zone réactionnelle à mélange presque parfait.

10. Utilisation selon la revendication 8 dans laquelle le catalyseur est mis en oeuvre en lit mobile à co-courant.

## Claims

1. Catalyst containing silica and an acid phase consisting of sulphuric acid and the compound HB(HSO₄)₄, the silica having been impregnated with said acid phase and said catalyst being such that it is essentially composed of particles with an average diameter of between 0.1 and 150 µm, in that its content by weight of acid phase is greater than 40%, in that the silica, prior to its impregnation with said acid phase, has a total porous volume of between 0.5 and 6 cm³ per gram, in that said acid phase contains (in % by weight) between 0.4% and 68.8% of the compound HB(HSO₄)₄ and between 31.2% and 99.6% of the compound H₂SO₄, said acid phase being characterised by the fact that it does not contain sulphuric anhydride or boric acid.

2. Catalyst according to claim 1 such that it is essentially constituted by particles of average diameter of between 5 and 110 µm.

3. Catalyst according to one of claims 1 or 2, such that the silica, prior to its impregnation with said acid phase, has a total porous volume of between 0.6 and 6 cm³ per gram.

4. Catalyst according to one of claims 1 to 3, such that the silica, prior to its impregnation with said acid phase, has a total porous volume of between 1.5 and 6 cm³ per gram.

5. Catalyst according to one of claims 1 to 4 such that the content by weight of said acid phase is greater than 70%.

6. Preparation of the catalyst according to one of claims 1 to 5 containing at least two steps, the first step being such that the silica is calcined at a temperature greater than 50°C for a duration of between 10 minutes and 50 hours, and the second step being such that said calcined silica is impregnated by said acid phase.

7. Preparation according to claim 6 in which the compound HB(HSO₄)₄ is obtained by reacting 1 mol of boric acid with 3 mols of sulphuric anhydride and 1 mol of sulphuric acid.

8. Use of the catalyst according to one of claims 1 to 5 or prepared according to one of claims 6 or 7 in catalytic alkylation of at least one isoparaffin selected from the group formed by isobutane and isopentane in the presence of at least one olefin containing 3 to 6 carbon atoms per molecule, in which the reaction temperature is lower than +10°C and the pressure of the reaction zone is sufficient to maintain the hydrocarbons in liquid state in said zone.

9. Use according to claim 8 in which the catalyst is used in a near-perfect mixture reaction zone.

10. Use according to one of claim 8 in which the catalyst is used in a co-flow fluidised bed.

## Patentansprüche

1. Katalysator, Silicium(di)oxid und eine saure Phase umfassend, die Schwefelsäure und die Verbindung HB (HSO₄)₄ aufweist, wobei das Silicium(di)oxid durch diese saure Phase imprägniert ist, der Katalysator derart ist, daß er im wesentlichen aus Partikeln eines mittleren Durchmessers zwischen 0,1 und 150 µm gebildet ist, daß sein Gewichtsgehalt an saurer Phase über 40 % liegt, daß das Silicum(di)oxid vor seiner Imprägnierung mit dieser sauren Phase ein Porentotalvolumen zwischen 0,5 und 6 cm³/g besitzt, daß diese saure Phase (in Gew.-%) zwischen 0,4 % und 68,8 % der Verbindung HB (HSO₄)₄ und zwischen 31,2 % und 99,6 % der Verbindung H₂SO₄ besitzt, wobei diese saure Phase sich dadurch auszeichnet, daß sie kein Schwefelsäureanhydrid und auch keine Borsäure enthält.

2. Katalysator nach Anspruch 1, der im wesentlichen aus Partikeln von einem mittleren Durchmesser zwischen 5 und 110 µm gebildet ist.

3. Katalysator nach einem der Ansprüche 1 oder 2, von der Art, daß Silicium(di)oxid vor seiner Imprägnierung durch diese saure Phase ein Porengesamtvolumen zwischen 0,6 und 6 cm³/g besitzt.

4. Katalysator nach einem der Ansprüche 1 bis 3, von der Art, daß das Silicium(di)oxid, vor seiner Imprägnierung durch diese saure Phase, ein Porengesamtvolumen zwischen 1,5 und 6 cm³/g besitzt.

5. Katalysator nach einem der Ansprüche 1 bis 4, von der Art, daß der Gewichtsgehalt an dieser sauren Phase über 70 % beträgt.

6. Herstellung des Katalysators nach einem der Ansprüche 1 bis 5, wenigstens zwei Stufen umfassend, wobei die erste Stufe von der Art ist, daß das Silicium(di)oxid bei einer Temperatur oberhalb 50°C über eine Dauer zwischen 10 Minuten und 50 Stunden kalciniert wird und die zweite Stufe von der Art ist, daß das kalcinierte Silicum(di)oxid durch diese saure Phase imprägniert wird.

7. Herstellung nach Anspruch 6, bei der die Verbindung HB (HSO₄)₄ erhalten wird, indem man 1 Mol Borsäure mit 3 Molen Schwefelsäureanhydrid und 1 Mol Schwefelsäure reagieren läßt.

8. Verwendung des Katalysators nach einem der Ansprüche 1 bis 5 oder hergestellt nach einem der Ansprüche 6 oder 7, um der katalytischen Alkylierung wenigstens eines Isoparaffins, das gewählt ist aus der Gruppe, die gebildet wird durch Isobutan und Isopentan in Anwesenheit wenigstens eines Olefins, das 3 bis 6 Kohlenstoffatome pro Molekül umfaßt, wobei die Reaktionstemperatur unterhalb +10°C und der Druck der Reaktionszone ausreichend ist, um die Kohlenwasserstoffe im flüssigen Zustand in dieser Zone zu halten.

9. Verwendung nach Anspruch 8, bei der der Katalysator in einer Reaktionszone in fast vollkommener Mischung eingesetzt wird.

10. Verwendung nach Anspruch 8, bei der der Katalysator im beweglichen Bett im Gleichstrom eingesetzt wird.
